# EUROPEAN PATENT SPECIFICATION

(11) **EP 2 971 031 B1**
(45) Date of publication and mention of the grant of the patent: **29.05.2019**
(21) Application number: 14764417.3
(22) Date of filing: 15.03.2014
(51) Int. Cl.: C12P 19/34

(54) **APPARATUS FOR RAPIDLY AND CYCLICALLY HEATING AND COOLING A FLUID SAMPLE DURING PCR TESTING**
VORRICHTUNG ZUM SCHNELLEN ZYKLISCHEN ERHITZEN UND KÜHLEN EINER FLÜSSIGEN PROBE WÄHREND EINES PCR-TESTS
APPAREIL PERMETTANT DE CHAUFFER ET DE REFROIDIR D'UNE MANIÈRE RAPIDE ET CYCLIQUE UN ÉCHANTILLON DE FLUIDE PENDANT UN ESSAI DE PCR

(30) Priority: 15.03.2013 US 201361792855 P
(43) Date of publication of application: 20.01.2016
(73) Proprietor: Drummond Scientific Company, Broomall, PA 19008 (US)
(72) Inventor: DRUMMOND, David, Albuquerque, NM 87120 (US); STROHSAHL, Christopher, Chesterbrook, PA 19087 (US)
(74) Representative: Manitz Finsterwald Patent- und Rechtsanwaltspartnerschaft mbB
(86) International application number: PCT/US2014/030002
(87) International publication number: WO 2014/145268

(56) References cited:
- EP-A1- 2 145 949
- WO-A1-2007/087690
- WO-A1-2007/091230
- WO-A2-2009/006447
- US-A1- 2002 055 149
- US-A1- 2010 129 872
- US-A1- 2012 021 463
- US-B1- 6 534 009
- P.-A Auroux ET AL: "Sample-Shunting Based PCR Microfluidic Device", , 1 January 2004 (2004-01-01), pages 67-69, XP055289566, Retrieved from the Internet: URL:http://nsti.org/publications/Nanotech/ 2004/pdf/B1-17.pdf [retrieved on 2016-07-19]
- AUROUX ET AL.: 'PCR Micro-Volume Device for Detection of Nucleic Acids' NANOTECH vol. 1, 2003, pages 1 - 4, XP055289520 Retrieved from the Internet: <URL:www.nsti.org>
- AUROUX ET AL.: 'Sample-Shunting Based PCR Microfluidic Device' NSTI-NANOTECH vol. 1, 2004, pages 67 - 69, XP055289566 ISBN: 0-9728422-7-6 Retrieved from the Internet: <URL:www.nsti.org>

## Description

### Field of the Invention

The present application relates to an apparatus for rapidly and cyclically heating and cooling a fluid sample during laboratory or clinical testing. In particular, the method and apparatus are useful during polymerase chain reaction (PCR) diagnostic procedures.

### Background of the Invention

Polymerase chain reaction (PCR) is a process that uses primers to amplify specific cloned or genomic DNA sequences with the help of a unique enzyme. The polymerase chain reaction is used in medical and biological research labs for a variety of applications including DNA cloning or sequencing, DNA-based phylogeny, forensic and paternity testing, and most genetic diagnostic testing. Using PCR, a chosen DNA segment is replicated every time the sample fluid is processed through a predetermined thermal cycle, allowing for amplification of the sample concentration by several orders of magnitude. The speed at which the sample completes the thermal cycle is a major factor in determining the time required to perform the genetic assay. During the PCR procedure, the progress of the reaction, or the amount of the product produced, can be monitored by measuring the fluorescence from molecular beacon probes, which are engineered to fluoresce at low temperatures only when bound to the target DNA sequence.

In the prior art, PCR is commonly carried out using small reaction tubes, typically 0.2-0.5 ml volumes, in a thermal cycler. The reaction volume is typically 10-200µl. The reaction tubes are inserted into bores within the thermoelectric block, which heats and cools the reaction tubes in discrete, pre-programmed steps. Because the reaction tubes are heated and cooled by conduction through the tube walls, heat transfer to and from the DNA target is slow and often takes several hours to complete.

It is also known to carry out PCR using closed-end, small-volume (20 µl) capillary tubes, which are heated and cooled using a forced-air thermal cycler, as in the Roche Pharmaceuticals LightCycler machine. Because the capillary tube wall is very thin (1.5mm OD, 1.1mm ID), heat transfer to and from the DNA target is much faster than in a traditional thermal cycler. As a result, the duration of a full amplification procedure can be reduced to about half an hour. However, it would be desirable to reduce the duration of a full amplification cycle even further, which in turn would reduce the cost, and increase the utility, of PCR testing.

WO 2009/006447 A2 discloses a bioanalysis, liquid processing system which includes target solution tubes (110) in contact with heating blocks (252, 254, 256, 258) that are set at specific heating temperatures. Pistons assemblies (120) engage a first end of the tubes (110). Liquid aliquots or slugs 130 are admitted to the second open ends of the tubes (110). Movement of the piston assemblies causes the aliquots to travel from one heated section of the tube to another, at which the aliquots are heated by conduction to a different temperature.

WO 2007/091230 A1 discloses a continuous, serpentine tube 10 through which target solution flows. The upper, middle and lower portions of the tube contact three different thermal zones (11, 12, 13), which are controlled to achieve the three individual temperature zones required for a PCR reaction. The solution is driven through the tube at a slow, steady velocity.

A. Auroux et al., "Sample-Shunting Based PCR Microfluidic Device," retrieved from http://nsti.org/publications/Nanotech/2004/pdf/B1-17.pdf, pp. 67-69, 1 January 2004 (XP055289566) discloses a micro thermal cycler using a solution channel passing through three temperature zones. Amplification detection and testing is carried out between cycles. D3 teaches that PCR samples were cycled between the three temperature zones using a pump.

US 2010/129872 A1 discloses a reaction vessel having two heat exchanger zones. A pump adjusts the pressure within the vessel to position a reaction mixture within the vessel as it moves between zones. The position of the mixture is detected by an optical sensor. The degree of amplification is analyzed between trials.

### Summary of the Invention

Using the apparatuses of the present invention, the duration for full amplification of target DNA using most PCR procedures is greatly reduced compared to the prior art. Instead of heating and cooling static target solution in a reaction tube, the target solution reciprocates (alternately flows) back and forth within an elongate, thick-walled, small-bore tube. Sections of the tube are maintained at different temperatures so that an ascending/descending temperature gradient is maintained along the length of the tube. As the target solution flows within and along the tube from section to section, it rapidly achieves thermal equilibrium with the tube at each section, thereby rapidly thermally cycling the target solution.

The apparatus of the present invention as claimed in claim 1 has numerous advantages compared to prior art PCR thermal cycling devices. Most notably, the apparatus enables standard PCR procedures to be conducted far more quickly than prior art devices without sacrificing accuracy or reliability.

Because the target solution is shuttled back and forth within the reaction tube, rather than sitting statically in the reaction tubes of the prior art, the sample is heated and cooled much more quickly, thereby shortening the overall thermal cycle. By shuttling the target solution back and forth within the reaction tube, the solution is heated by conduction and convection. Thermal conduction occurs from contact between the target solution and the interior wall of the reaction tube. Convection occurs from internal stirring or mixing of the target solution, which is induced from laminar flow through the tube. As the target solution flows through the tube, rapid fluid flow patterns are created between the solution near the tube wall and the solution in the middle of the tube.

The sample target solution is also heated and cooled much more quickly compared to the prior art since the apparatus does not thermally cycle the reaction tube along with solution contained therein. Instead, the sample is shuttled back and forth to different zones of reaction tube, each of which is maintained at a different temperature. Since the thermal capacity of solution is far less than the combined thermal capacity of the solution and reaction tubes of the prior art, far less energy is required to thermally cycle the solution using the apparatus and method described herein. These energy savings are not completely negated by the energy required to reciprocate the target solution within the reaction tube.

The sample target solution is also heated and cooled much more quickly compared to the prior art since the thick-walled, small-bore reaction tube of this apparatus has a much higher heat capacity than thin-wall reaction tubes of the prior art. The increased thermal conductivity properties of reciprocating solution would be greatly reduced if the heat capacity of the tube was not increased proportionately compared to the prior art.

The sample target solution is also heated and cooled much more quickly compared to the prior art since the target solution contacts a much greater tube wall surface area as measured on a solution unit volume per tube unit area basis. The reaction tube of the apparatus has a very small cross-section and very long length compared to prior art reaction tubes. As a result, given the same sample volume, the target solution contacts and is heated/cooled by a far greater reaction tube surface area.

The apparatus maintains the sterility of subsequent test samples since the reaction tube is disposable. Additionally, the pressure injector may include a disposable syringe, which is the only component of the pressure injector that could possibly come into contact with the target solution. Therefore, after each procedure, every component that could contact the target solution, even accidentally, is replaced with a new, sterile component.

The apparatus of the present invention includes a component that measures amplification progress in real-time. During each thermal cycle, the unique amplification monitor of the apparatus detects and measures the amount of fluorescence from molecular probe molecules within the target solution. Using experimentally derived data, the fluorescence level can be correlated to amplification level for a particular DNA target and target solution.

In accordance with one preferred embodiment, the apparatus for performing a polymerase chain reaction (PCR) procedure on a target DNA solution includes an elongate tube having a proximal end, a distal end, lengthwise axis, denaturation zone, annealing zone, and elongation zone. The tube is preferably thick-walled and has a small bore wherein the ratio of the tube outer diameter to the tube inner diameter is greater than about 4 to 1.

Means are provided for heating the three heating/cooling zones at independent, elevated temperatures. In a preferred embodiment, the heating means comprises a thermoelectric heating block in each zone. The blocks contact and support the tube relative to a work surface. Each block includes an elongate groove in which the tube is seated. The heating/cooling blocks maintain each of the denaturation, annealing and elongation zones at a temperature that permits PCR denaturation, annealing and elongation, respectively.

Means are provided for shuttling the target solution back and forth within the tube from one zone to another in a repeating thermal cycle. In one preferred embodiment, the shuttling means comprises pneumatic drive means such as a pressure injector connected to one end of the tube. The pressure injector preferably includes a disposable syringe that is connected in fluid communication with the tube.

Means are also provided for controlling the movement and the axial position of the target solution within the tube during the thermal cycle. The control means includes means for detecting the position of the target solution within each heating/cooling zone. In one embodiment, the position detecting means comprises a laser emitter and detector arranged in an alignment to project a laser beam through the tube at an angle transverse to the lengthwise axis of the tube. Preferably a plurality of laser emitters and detectors are arranged in alignments that project a laser beam through each zone of the tube at an angle transverse to lengthwise axis of the tube.

In another preferred embodiment, the apparatus includes means for detecting the level of amplification of the target DNA within the tube. Preferably, the detecting means detects the level of amplification of the target DNA during each thermal cycle. In a preferred embodiment, the detecting means comprises a plurality of optical fibers arranged in a linear array in close proximity to and coaxial with the tube in the annealing zone.

In another embodiment of the invention, the apparatus for performing a polymerase chain reaction (PCR) procedure on a target DNA solution comprises a reaction container having separate denaturation, annealing and elongation heating/cooling zones; means for heating three heating/cooling zones at independent, elevated temperatures; means for shuttling the target solution back and forth within the container from one zone to another in a repeating thermal cycle; means for conductively and convectively heating/cooling the target solution in each zone; and, means for controlling the movement and position of the target solution within the tube during the thermal cycle.

Using an apparatus according to the invention, an unclaimed method for performing a polymerase chain reaction (PCR) procedure on a target DNA solution having a DNA target and PCR primers and reagents comprises the steps of introducing the target solution into a reaction tube, and thermally cycling the target solution within the tube by heating and cooling the target solution by simultaneous conduction and convection to a denaturation temperature, annealing temperature, and an elongation temperature. The target solution is cyclically heated to at least the melting temperature of the DNA target during a first phase of the thermal cycle, then cooled to a temperature below the melting temperature of the primers of the specific reaction during a second phase of the thermal cycle, then heated to about the optimum activity temperature of the DNA polymerase used in the target solution during a third phase of the thermal cycle.

The method may include the steps of maintaining the temperature of at least three different heating/cooling zones of the tube at separate elevated temperatures, and transporting the solution back and forth between all three zones and dwelling the fluid at all three zones for a predetermined duration. Preferably, internal mixing of the solution is induced at each of the zones.

Preferably, the solution flows through the container from one zone to another. The solution may flow by creating a pressure differential across the solution.

In another method, the amplification level of the target DNA is measured during each thermal cycle. Amplification may be measured by, for example, measuring the fluorescence of probe molecules within the target solution during the annealing phase of the thermal cycle.

### Brief Description of the Drawings

Fig. 1 is a schematic illustration of an apparatus for performing a PCR procedure in accordance with an embodiment of the invention;
Fig. 2 is a top plan view of an apparatus for performing a PCR procedure in accordance with an embodiment of the invention showing the top cover in a retracted position;
Fig. 3 is a top plan view of the apparatus of Fig. 2 showing the top cover in a closed position;
Fig. 4 is an enlarged, perspective view of the proximal end of the apparatus of Fig. 2 showing the amplification monitor;
Fig. 5 is an enlarged perspective of a heating block of the apparatus of Fig. 2;
Fig. 6 is a side elevation of the coupler, reaction tube and end cap of the apparatus of Fig. 2;
Fig. 7 is an enlarged, cross-sectional view of the tube coupler;
Fig. 8 is an enlarged, cross-sectional view of the tube coupler and the proximal end of the reaction tube connected thereto;
Fig. 9 is a cross-sectional view taken long lines 9-9 of Fig. 8; and
Fig. 10 is an enlarged, cross sectional view of the tube illuminator of Fig. 2.

### Detailed Description of Preferred Embodiments

For the purpose of illustrating the invention, an embodiment of the invention is shown in the accompanying drawings. However, it should be understood by those of ordinary skill in the art that the invention is not limited to the precise arrangements and instrumentalities shown therein and described below. Throughout the specification, like reference numerals are used to designate like elements. Numerous changes and modifications within the scope of the invention will become apparent to those skilled in the art from this detailed description.

Unless otherwise defined, all technical and scientific terms used herein in their various grammatical forms have the same meaning as commonly understood by one of ordinary skill in the art to which this invention belongs. The term "melting temperature" when used in connection with DNA or DNA target means the temperature at which a DNA double helix dissociates into single strands. The term "melting temperature" when used in connection with a PCR primer means the temperature at which 50% of that same DNA molecule species form a stable double helix and the other 50% have been separated to single strand molecules. The term "DNA target" means a specific region of a DNA strand that is to be amplified. The term "target solution" means the solution containing the DNA target and all the required PCR reagents including, but not limited to, primers and polymerase. With reference to an elongate element or series of elements, the term "proximal" means the element or portion of element closest to the tube illuminator, and the term "distal" means the element or portion of element farthest from the tube illuminator.

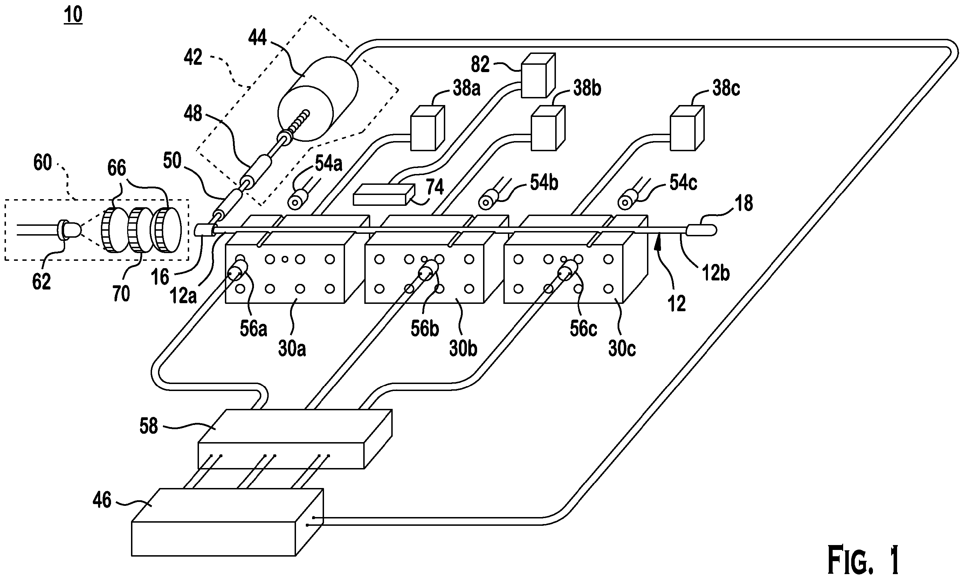

An apparatus for performing a polymerase chain reaction (PCR) procedure on a target DNA solution in accordance with an embodiment of the invention is illustrated in Figs. 1-13 and is designated generally by reference numeral 10. In general, the apparatus includes an elongate reaction tube, means for heating and cooling separate zones (sections) of the tube at different temperatures, pneumatic pressure means for shuttling the target solution back and forth within the reaction tube to each heating/cooling zone, and control means for thermally cycling the target solution at a pre-programmed sequence. Depending on the particular PCR procedure and target DNA, the temperature of each zone, dwell time at each zone, and number of cycles will vary.

The apparatus, designated generally by reference numeral 10, is schematically illustrated in Fig. 1. In a preferred embodiment, the elongate reaction tube 12 is oriented horizontally relative to its elongate axis and rests on thermal blocks 30. The tube 12 has a proximal end 12a, which is connected to the pneumatic pressure means by a coupler 16, and a distal end 12b, which has a removable end cap 18. A small sample (column) of target solution can be admitted to the tube 12 by removing the cap 18, and admitting the fluid either by capillary action, external injection, or internal suction.

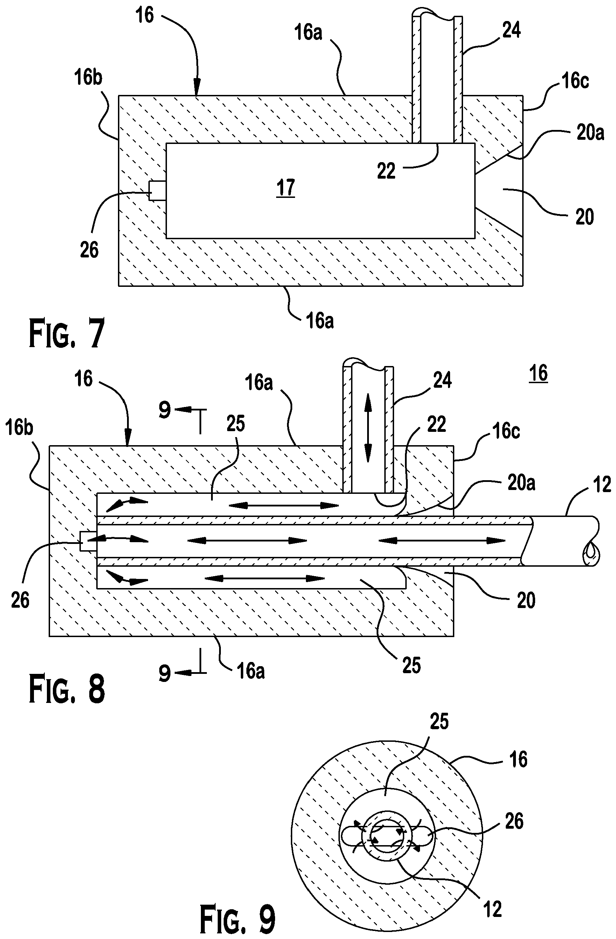

Referring to Figs. 7-9, the coupler 16 has a generally cylindrical shape with an axially extending side wall 16a and radially-extending end walls 16b, 16c, which define an internal cavity 17. The coupler is preferably made from an elastic material such as clear silicone. The coupler material is also preferably transparent so that it can transmit light from the tube illuminator 60 through the tube 12. A first port 20 extends axially through the distal end wall 16c. A second port 22 extends radially through the side wall 16a.

The first port 20 has a frusto-conical shape with side walls 20a that taper radially outwardly from inside to outside so that the tube 12 can be easily inserted into the first port. As seen in Fig. 7, the inner diameter of the first port 20 is smaller than the outer diameter of the tube. A good, temporary seal is formed between the coupler 16 and the tube 12 because of this interference fit and the elastomeric properties of the coupler 16.

The second port 22 has a cylindrical shape with side walls that engage the outer surface of a tubular stem 24 that connects to the pneumatic pressure source 42. The inner diameter of the second port 22 is preferably smaller than the outer diameter of stem 24. A good, temporary seal is formed between the coupler 16 and the tube stem 24 because of this interference fit and the elastomeric properties of the coupler 16.

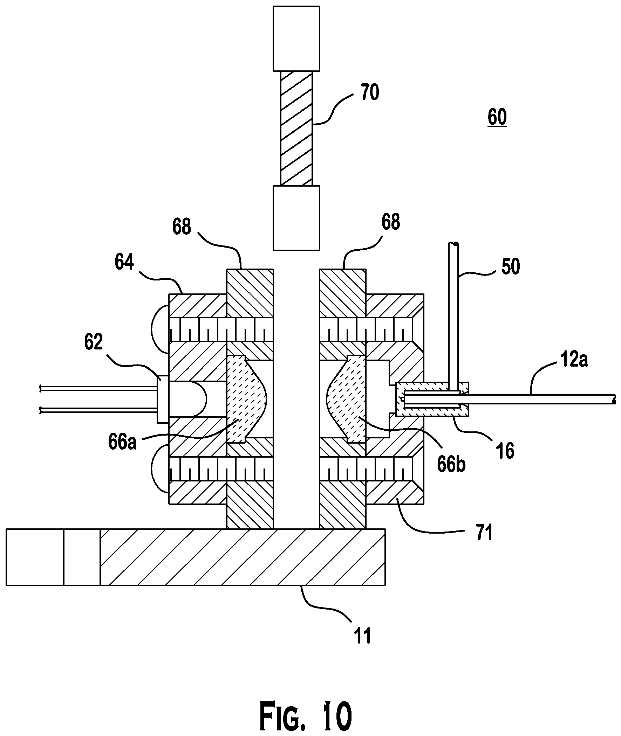

Referring to Fig. 10, the proximal end of the coupler is fixed to a tube illuminator 60. In order to properly propagate light through the tube and illuminate the target solution contained therein, the outer surface of the cylindrical tube wall must not contact the inner surface of the side wall 16a; otherwise light will escape at such contact points. Furthermore, the proximal end face of the tube 12 should contact the inner surface of the proximal end wall 16b. Light is propagated along the tube 12 by total internal reflection. Therefore, in order to create a fluid flow pathway from the pneumatic pressure source 42 through the tube 12, the diameter of the inner cavity 17 is larger than the outer diameter of the tube 12, which forms an axial fluid flow gap 25 between the connector 16 and tube 12. Furthermore, an airflow groove 26 is formed in the inner surface of the proximal end wall 16b. As best seen in Figs. 7 and 8, a fluid flow pathway extends from the stem 24, through the axial fluid flow gap 25, through the air flow groove 26, and then through the inner tube 12.

The reaction tube preferably comprises a rigid, elongate, thin-bore capillary tube made of a material that is inert to the target solution reagents. In a preferred embodiment, the tube is made from thick-walled, small-bore polycarbonate. While glass has better optical transmission properties than polycarbonate, glass is less desirable for facilitating the amplification process.

The dimensions of the inner bore and wall thickness of the tube 12 should be selected so that the tube has a thick wall compared to the inner bore. In a preferred embodiment, the tube material and dimensions are selected so that the heat capacity (C_{T}) of the tube wall per unit volume is much greater than the heat capacity (C_{S}) of the target solution per unit volume flowing therethrough. As used herein, the heat capacity (C_{T}) of the tube is equal to the specific heat capacity (specific heat) of the tube material per unit volume multiplied by the unit volume of the tube wall. The heat capacity (C_{S}) of the target solution is equal to the specific heat capacity (specific heat) of the target solution multiplied by the unit volume of target solution within the inner bore of the tube. Preferably C_{T}/C_{S} should be greater than about 4.

In one preferred embodiment, the tube is made from polycarbonate and has a 0.014 in. (0.55 mm) inner diameter and a 0.060 in. (0.24 mm) outer diameter. The cross-sectional area of the tube wall is about sixteen times the cross-sectional area of target solution in the inner bore. However, the specific heat of the wall material (polycarbonate) is about 33% of the specific heat of the target solution (similar to water). Therefore, the heat capacity of the tube is about 4.8 times that of the target solution.

As explained below, thick-walled, small-bore tubing enables the target solution to reach thermal equilibrium with the tube much more quickly than a thin walled tube. In the preferred embodiment illustrated herein, the aforementioned ratio of heat capacities enables the target solution to equalize at a temperature within about 5°C of the block temperature when the temperature difference between blocks is about 30°C.

The length of the tube will vary depending on the intended volume of target solution to be processed per procedure. To minimize the amount of time needed to reach thermal equilibrium with the tube, and to ensure proper heating of the entire column of target solution during each phase of the thermal cycle, the tube length in each zone should be larger than the solution column length. This relative sizing also contributes to the overall disparity in heat capacity between the tube and target solution since the target solution can absorb heat from the unoccupied portion of the tube as well as the occupied portion.

Preferably, the solution column length is about 80% to about 90% of the tube length within each heating/cooling zone. In one preferred embodiment illustrated herein, the total tube length is about 7.5 in. (19.1 cm) long. As described below, when used in combination with 3 heating blocks, each being 2 in. (5.01 cm) long, the tube length per heating zone is about 2 in. (5.1 cm), which correlates to a fluid column sample of about 1.6 in. (6.3 mm) to about 1.8 in. (7.1 mm). Using the above-described parameters, when the target solution of temperature T_{S} reaches thermal equilibrium with a wall section of temperature of T_{T}, their temperature is about 5°C of the block temperature T_{B}.

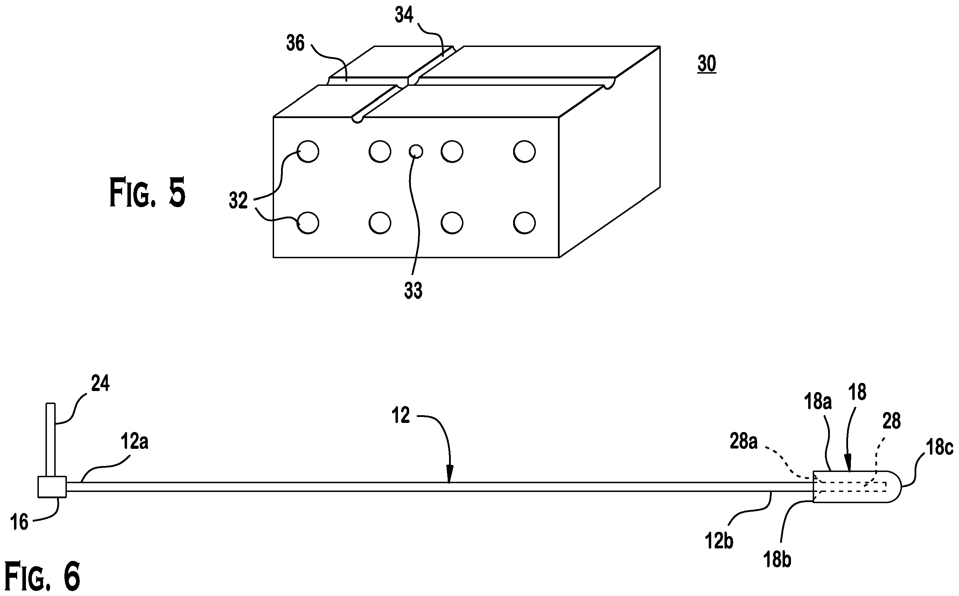

Referring to Fig. 6, the end cap 18 has a generally cylindrical shape with an axially extending side wall 18a and radially-extending end walls 18b, 18c, which define an internal cavity. The end cap 18 is preferably made from an elastic material such as clear silicone. A bore 28 extends axially through the proximal (relative to the illumination source described below) end wall 18b. Similar to the first port 20 of the coupler 16, the leading end 28a of the bore 28 has a frusto-conical shape with side walls that taper radially outwardly from inside to outside so that the tube 12 can be easily inserted into the bore 28. The inner diameter of the bore 28 is smaller than the outer diameter of the tube 12. A good, temporary seal is formed between the end cap 18 and the tube 12 because of this interference fit and the elastomeric properties of the cap 18. The end cap 18 can be installed and removed from the distal end of the tube 12 simply by pushing or pulling, respectively, the cap 18 over the end of the tube.

The end cap 18 prevents target solution from spilling out of the tube during the PCR procedure. In a preferred embodiment, the hottest zone of the tube is located proximally to the distal end 12b of the tube 12. The temperature at this zone can be as high as 98-100 °C. During thermal cycling (described below), the target solution has a tendency to expand rapidly and squirt outwardly as it enters this "hot" zone due to the sudden water vapor pressure increase as the target solution approaches its boiling point. The cap 18 acts a physical barrier to target solution expulsion.

The cap 18 also functions as a pressure cap. As the target solution is propelled from the proximal end 12a to the distal end 12b of the tube 12, the column of air in the tube ahead of the target solution column (distal side) is compressed, causing an increase in pressure within the tube 12. In the embodiment shown in Figs. 1-10, the pressure in the tube 12 increases to about 2 atmospheres. As a result, the boiling point of the target solution increases as the pressure increases. With an increased boiling point, the target solution can be heated to higher temperatures than possible with an open-ended tube.

In a preferred embodiment shown in Figs. 1-10, the heating elements comprise thermoelectric heating blocks 30. However, it should be appreciated that other heating elements could be used without departing from the invention. For example, the heating elements could comprise forced air heaters, forced fluid heat exchangers, radiant heaters, or electrically-resistive heaters, so long as the heating elements can maintain different, elevated-temperature zones along the length of the tube 12.

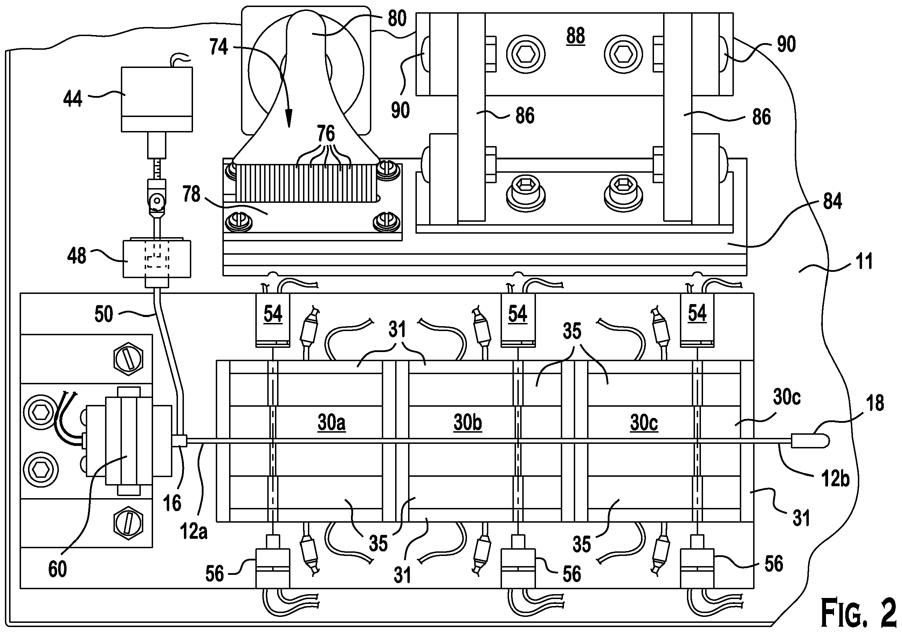

As best seen in Fig. 1, the reaction tube 12 rests on three heating blocks 30. Referring to Fig. 5, the blocks have a generally rectangular shape and a lengthwise-extending groove 36 in the top wall 30b as seen in Figs. 2-3. Each block sits in a housing 31, which includes a layer of insulation 35 surrounding the block 30. The blocks 30 are arranged in series so that their lengthwise-extending grooves are co-linear.

The groove 36 has a shape and size that compliments the shape and size of the bottom half (roughly) of the reaction tube so that reaction tube sits snugly and in good surface contact with the groove 36. This close, complimentary fit insures maximum heat transfer from the heating blocks 30 to the reaction tube 12. The section of tube in contact with the block 30 defines a heating/cooling zone.

Referring to Fig. 5, the heating blocks 30 include a plurality of widthwise-extending bores 32 in which thermo-electric, resistive heating elements are inserted. Referring to Fig. 1, the heating elements (not shown) of each block are connected to a separate controller 38a, 38b, 38c, which regulates the temperature of each block 30a, 30b, 30c, respectively, independent of one another. Each block 30 also includes a bore 33 in which a known temperature sensor or thermocouple (not shown) is inserted and connected to each controller 38a, 38b, 38c.

The blocks also have a widthwise-extending groove 34 in the top wall. As described below, the groove provides a passageway through which a photoelectric detector beam or laser beam may be projected. The widthwise groove 34 is arranged so that the detector beam projects directly transversely through the tube 12 and detects the presence of target solution at that location within the tube 12.

In a preferred embodiment, the blocks 30 are made from aluminum; however, the blocks may be made of any material that has a heat capacity substantially larger than the target solution. Aluminum, or other highly-reflective metals, is a useful block metal because of its optical reflectivity. The shiny surface of the tube channel 36 reflects the light which is emitted in a downward direction back up into the optical fibers of the amplification detector (described below).

In the embodiment shown in Figs. 1-10, the blocks 30 are about 2 in. (5.1 cm) long, 1 in. (2.54 cm) wide and 1 in. (2.54) high. At these dimensions, the blocks have a heat capacity of about 159 J/°C. In contrast, a common target solution has heat capacity of about .024 J/°C, which is negligible compared to the heat capacity of the blocks 30. This intended disparity of heat capacities minimizes the time required for the target solution to reach thermal equilibrium with the tube 12, and also insures that the heating blocks 30 are not thermally drained/cooled by the target solution below its intended operating temperature.

As described above, the overall length of the tube 12 will vary depending on the intended volume of target solution to be processed per procedure. Similarly, therefore, the size of the blocks 30 is based on the length of the tube and target solution test volume. Each block length is preferably about 1.1 to 1.25 times the column length of the target solution sample.

The length of the blocks 30 may also vary depending on the intended use of the apparatus 10. For example, if the intended use is strictly diagnostic, and the progress of the reaction is monitored in situ, the length of the heating blocks may be short. For applications where the sample will be analyzed post-amplification (e.g. sequencing), and a larger volume is necessary, then the heating blocks may be longer.

The apparatus 10 includes pneumatic drive means arranged in fluid communication with the proximal end of the reaction tube 12. The pneumatic drive means shuttles the column of target solution back and forth within the tube 12. In the embodiment shown in Figs. 1-10, the pneumatic drive means comprises a pressure injector 42, which is removably connected to the coupler stem 24 by a connection tube 50.

The injector 42 may comprise any device that is capable of controllably and cyclically creating negative and positive pressure in the tube 12 to alternately drive or propel the target solution back and forth along the length of the activation tube 12. Since the volume of the target solution within the reaction tube is small (usually about 5-6 µl), and the overall internal volume of the reaction tube is also small (about 20 µl) the injector 42 must be sensitive enough to control microliter amounts of fluid over short distances. The injector 42 must also have sufficient power to compress the air space ahead of the solution column as the column reaches its most distal heating zone. As described above, since the tube is sealed at the distal end, the air in the tube cavity on the distal side of the column become compressed to about 2 atmospheres as the column reaches the third zone, block 30c. The injector 42 must have sufficient power to overcome this force.

In the embodiment shown in Figs. 1-10, the injector comprises an auto-nanoliter injector sold by Drummond Scientific under the trademark Nanoject®. The injector generally comprises a stepper motor 44, which is connected to a controller 46. The stepper motor 44 reciprocates a replaceable/disposable syringe 48, which is removeably connected to the coupler stem via a fluid connection tube 50. The controller 46 operates the stepper motor 44 according to preprogrammed instructions and in response to signals from the solution position detector 60 (described below).

In a preferred embodiment, the pressure injector is connected to proximal end 12a of the tube 12. However, in another less preferred embodiment, the pressure injector maybe connected to the distal end 12b of the tube 12.

The apparatus includes means for detecting the position of the column of target solution within each heating/cooling zone. Once the position detector determines that the entire column of target solution is located with a heating/cooling zone, it signals the pressure injector controller 46, which commands the injector to stop reciprocation of the column for a preprogrammed delay or dwell period during which the solution undergoes one of the phases of the PCR procedure. At the end of the delay period, the controller 46 re-initiates reciprocation of the solution column until it is fully located in the next heating/cooling. This positioning function is repeated during each phase of the thermal cycle. In the preferred embodiment shown in Figs. 1-10, the position detector 60 comprises a plurality of laser beam emitters 52 and laser beam detectors 54. However, the position detector 60 may comprise any other suitable known detectors such as a variety of photoelectric sensors.

Each block 30a, 30b, 30c includes a laser beam emitter 52a, 52b, 52c and detector 54a, 54b, 54c, respectively. The emitters 52 are arranged to emit a laser beam through the widthwise groove 34 in each block 30. The detectors 54 are arranged on the opposite side of the emitters 52 in alignment with the laser beams. The laser beam is positioned to traverse the reaction tube 12. When the target solution crosses the path of the laser beam, it changes the intensity of the transmitted light by the beam, which is sensed by the detectors 54. At this moment, or after some preprogrammed or calculated delay, the position detector 54 signals the pressure injector controller 46 through a simple feedback loop. While the pressure injector 42 maybe preprogrammed with specific timing patterns, use of a position detector insures that the target solution receives the proper exposure at each heating/zone.

In a preferred embodiment, the lasers 54 detect the leading edge of the solution column. Therefore, the location of the widthwise grooves 32 and beam emitters 54 must account for the intended direction of travel of the solution column through a particular heating/cooling zone. Referring to Fig. 2, during the thermal cycle, the solution column starts at zone 1, block 30a, and then moves (to the right) to zone 2, block 30b, at which it rests for a preprogrammed duration. The laser emitter 54b and detector 56b of block 30b are located proximate the distal edge of the block. The distance of the laser emitter 54b and detector 56b should be selected so that the trailing edge of the solution column is also located within the heating/cooling zone, i.e., over the heating block 30b, when the leading edge traverses the laser beam.

For similar reasons, the emitter 54c and detector 56c on the distal block 30c is also located proximate the distal edge. However, after delaying in zone 3, block 30c, the solution column returns to zone 1, block 30a, travelling to the left as viewed in Fig. 2. Therefore, the emitter 54a and detector 56a are located near the proximal edge of the block 30a.

During thermal cycling, it is possible that a small amount of target solution may remain adhered to the reaction tube 12 in the laser beam path. The residual solution may cause an unintended interruption in the laser beam intensity and cause the detector to erroneously signal the pressure injector 42. In other preferred embodiments, the position detector 60 does not signal the pressure injector controller 46 until it senses passage of a predetermined amount of target solution. This delay is achieved by simply adjusting the sensor circuit and locating the widthwise-extending grooves 34, laser emitters 54 and detectors 56 farther away from the edge of the block 30.

In a preferred embodiment, the apparatus 10 includes means for monitoring DNA amplification of the target solution within the tube 12 in real time during the thermal cycle. The amplification monitor generally includes means for illuminating the activation tube and means for detecting fluorescence light exiting the target solution in the proximal heating/cooling zone at which annealing takes place.

Because the inner bore of the tube 12 is so small, the apparatus 10 transmits fluorescence exciting light into the target solution using the tubes internal reflection properties to carry the light down the walls of the tube 12. A tube illuminator, designated generally by reference numeral 60, focuses light on the open, proximal end 12a of the reaction tube 12. The light travels within the tube wall until it encounters the target solution. At that point, much of the light enters the liquid and excites fluorescence.

Referring to Fig. 10, the tube illuminator 60 includes an LED 62, which is mounted in an LED housing 64. Proximal 66a and distal 66b condenser lenses are mounted in lens housings 68 adjacent the LED. The lenses 66 condense and focus light down the length of the tube 12. A dichroic filter 70 is removably positioned in between the lenses to block unwanted wavelengths of light from the LED. The coupler is removably fixed in a holder 71, which is mounted flush against the distal lens 66b.

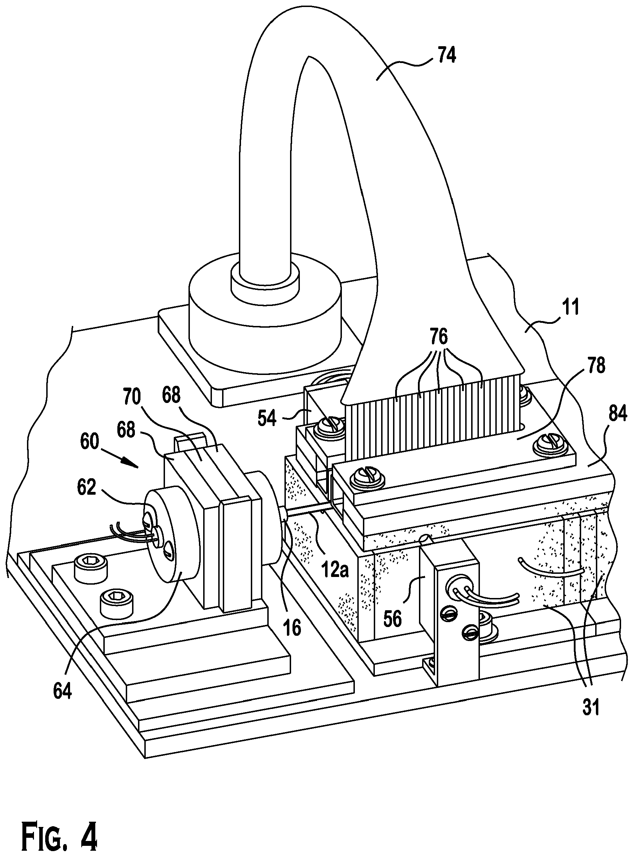

Referring to Figs. 2-4, fluorescence detector generally comprises a plurality of optical fibers 75 that are connected to a photomultiplier 82, and a retractable cover 84. As best seen in Figs. 4, one end of the fibers 75 is fixed in an alignment plate 78 in a linear array. The other end of the fibers 75 are arranged in a circular or hexagonal bundle by a sleeve 80, which configuration connects to the circular face of the photomultiplier tube.

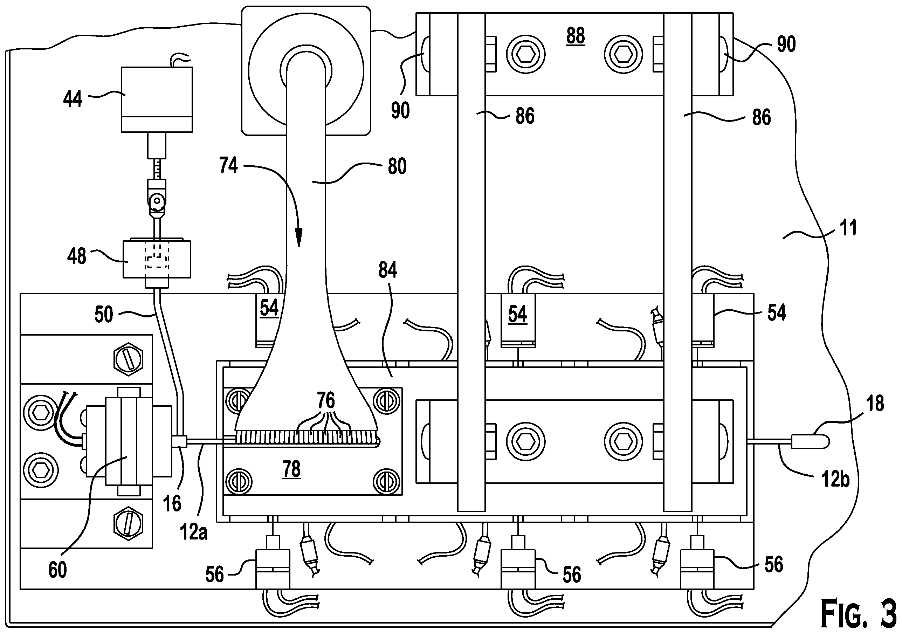

Referring to Figs. 2-3, the alignment plate 78 is fixed to the proximal end of the cover 84. The cover 84 has two arms 86 that are pivotally mounted to a base 88 by hinges 90. The detector base 88 is fixed to the base 11 of the apparatus 10. The cover 84 can be pivoted between a first (retracted) limit position shown in Fig. 2 and a second (closed) limit position shown in Fig. 3. In the retracted position, the operator has unobstructed access for installing the reaction tube on the heating blocks 30. In the closed position, the cover serves two functions. First, it provides thermal insulation to the top of the tube and above the heating blocks 30. Second, it aligns the linear array of optical fibers directly above the tube in zone 1, block 30a. While a portion of the light from the tube illuminator is directed down the tube 12 by total internal reflection, a larger percentage is radiated out the sides of the tube 12. At this location, the optical fibers detect the fluorescence intensity, which is directly proportional to the level of amplification in the target solution.

The optical array should be positioned over zone 1, block 30a, which is the coolest block and the location at which annealing takes place. To detect amplification, the target solution is infused with molecular beacon molecules. These molecules will fluoresce in the second and third zones, blocks 30b and 30c, whether or not they are attached to a DNA molecule; however, the beacon molecules will fluoresce in the first zone, block 30a, only if they are attached to one of the target DNA segments. Therefore, the intensity of the fluorescence is directly proportional to the level of amplification of the target DNA. Therefore, the optical array should measure fluoresce in the first zone, block 30a.

Herein is also described a novel method of performing a PCR procedure in less time than prior art PCR procedures. In accordance with the method, a target solution containing target DNA is prepared according to known procedures using known reagents. The target solution is then admitted to an elongate, thick-walled, small bore tube such as a capillary tube. In broad terms, the target solution is then thermally cycled by both conduction and convection within the tube. Conductive heat transfer to the target solution is achieved by heating the walls of the reaction tube and contacting the solution with the walls. Convective heat transfer to the target solution is achieved by shuttling the solution back and forth within the tube. Internal stirring or mixing of the target solution is induced from laminar flow through the tube. As the target solution flows through the tube, rapid fluid flow patterns are created between the solution near the tube wall and the solution in the middle of the tube.

Heat may be applied at several localized, axial locations of the tube, thereby creating heating/cooling zones. The zones are preferably maintained at different, elevated temperatures. One zone is maintained at a temperature at which denaturation will occur in the target solution, typically 90-100 °C. A second zone is maintained at a temperature at which annealing will occur in the target solution, typically 45-65 °C. A third zone is maintained at a temperature at which extension/elongation will occur in the target solution, typically 65-75 °C. Instead of thermally cycling a static volume of target solution, the solution is shuttled back and forth to each zone in a defined cycle. The target solution reaches thermal equilibrium with the tube within milliseconds of entering a particular zone. The target solution then dwells in each zone for a predetermined duration depending on the parameters of the particular PCR reaction.

The target solution may be shuttled back and forth within the tube using pneumatic pressure applied to at least one side of the solution column. Negative and positive pressure is alternately applied to one end of the reaction tube, thereby creating alternating pushing and pulling forces on the column of solution.

The pressure on the target solution is increased during at least one phase of the thermal cycle. Preferably, the pressure on the solution is increased in the hottest zone in which denaturation takes place. By increasing the pressure, the solution can be heated to a temperature higher than its atmospheric (normal) boiling point, which is especially helpful for conducting PCR procedures at high altitudes.

A preferred PCR method is described below using the apparatus 10 illustrated in Figs. 1-10. Initially, the heating blocks 30 are set at their predetermined temperature and the pressure injector 42 is pre-programmed with a specific thermal cycle. Next, the end cap 18 is removed and a small volume (about 5 µl) of target solution is admitted by injection or suction. The sample is then drawn to zone 1, which is section of the tube 12 in contact with the proximal heating block 30a. The distal end 12b of the tube is then re-sealed with the end cap 18.

From zone 1, the solution is shuttled to zone 3, which is the section of tube in contact with the distal heating block 30c. The target solution dwells in zone 3 for a predetermined duration for initial denaturation. The target solution is then shuttled back to zone 1 and dwells there for a predetermined duration for initial annealing. Next, the target is shuttled from zone 1 to the zone 2, which is the section of tube in contact with the middle heating block 30b. After dwelling in zone 2 for a predetermined duration, the solution is shuttled back to zone 3. The solution is thermally cycled from zone 3 to zone 1 to zone 2 and back to zone 3. The cycle consists of three phases, namely, advancing to and dwelling at each of the three heating/cooling zones.

The number of thermal cycles will vary and depends on the specific target solution and the desired level of amplification. The dwell time in each zone will also vary and depends on the specific target solution and the desired level of amplification. Extension (amplification) occurs in the second zone, which is usually the longest phase. In general, the ratio of dwell time in zone 2 compared to the dwell times in zones 1 and 3, which are generally equal, is usually about 2 to 1, but may be as great as 4 to 1 or as low as 1 to 1.

Using the apparatus 10 described above, the target solution reaches thermal equilibrium with the tube in each zone in about 300 to 400 milliseconds, which is far less than the time needed to reach thermal equilibrium in prior art thermal cyclers. The additional step (compared to the prior art) of shuttling the solution from one zone to another has little effect on the overall duration of the thermal cycle since the apparatus 10 shuttles the solution column from one zone to another in about 300 milliseconds.

Using the apparatus 10 described above, the air column on the distal side of the column is compressed and pressurized to about 2 atmospheres as the solution column reaches zone 3. Because of the increased pressure, the boiling point of the solution is increased, which allows the solution to be heated to temperatures higher than the boiling point at that particular location. For example, at an altitude of about 5,600 feet, experiments show that it is difficult to raise the temperature of some, un-pressurized target solutions above 90°C. However, the generally accepted denaturation temperature is 94-98 °C. The increased internal pressure created by the sealed reaction tube raises the boiling point of the target solution above the recommended denaturation temperature at such high-altitude testing facilities.

## Claims

1. An apparatus for performing a polymerase chain reaction (PCR) procedure on a column of target DNA solution, comprising:
a) an elongate tube (12) having a proximal end (12a), a distal end (12b), lengthwise axis, a denaturation zone, annealing zone, and elongation zone;
b) means for heating said three heating/cooling zones (30a, 30b and 30c) at independent, elevated temperatures;
c) means for shuttling (42) the target solution back and forth within said tube (12) from one zone to another in a repeating thermal cycle;
**characterized in that** it includes:
d) means for controlling (46) the movement and the axial position of the target solution within said tube (12) during the thermal cycle including means for detecting a position of the target solution within each said heating/cooling zone,
**and in that** the distal end (12b) of said elongate tube is sealed with a removable end cap (18).

2. The apparatus recited in claim 1, including means for conductively and convectively heating/cooling (30) the target solution in each zone.

3. The apparatus recited in any one of claims 1 and 2, wherein the ratio of the tube (12) outer diameter to the tube (12) inner diameter is greater than about 4 to 1.

4. The apparatus recited in any one of claims 1 and 2, wherein said heating means comprises a thermoelectric heating block (30) in each zone, contacting and supporting tube (12) relative to a work surface, and wherein each block (30) includes an elongate groove (36) in which the tube (12) is seated.

5. The apparatus recited in any one of claims 1 and 2, wherein said shuttling means (42) comprises pneumatic drive means (44).

6. The apparatus recited in claim 5, wherein said shuttling means (42) comprises a pressure injector (42) including a disposable syringe (48) connected in fluid communication to one end of said tube (12).

7. The apparatus recited in any one of claims 1 and 2, wherein said control means (46) includes means for detecting the position of target solution (60) within each heating/cooling zone.

8. The apparatus recited in claim 7, wherein said position detecting means (60) comprises a laser emitter (54) and detector (56) arranged in an alignment to project a laser beam through the tube (12) at an angle transverse to the lengthwise axis of said tube (12).

9. The apparatus recited in claim 8, including a plurality of laser emitters (54) and detectors (56) arranged in alignments that project a laser beam through each zone of the tube (12) at an angle transverse to lengthwise axis of the tube (12).

10. The apparatus recited in any one of claims 1 and 2, further including detecting means (76) that detects the level of amplification of the target DNA during each thermal cycle in real time.

11. The apparatus recited in claim 10, wherein each of the denaturation, annealing and elongation zones are maintained at a temperature that permits PCR denaturation, annealing and elongation, respectively.

12. The apparatus recited in claim 11, wherein said detecting means comprises a plurality of optical fibers (76) arranged in a linear array in close proximity to and in transverse axially alignment with the tube (12) in the annealing zone.

13. The apparatus recited in claim 1 wherein the tube (12) material and dimensions are selected such that the heat capacity of the tube (12) wall per unit volume (Ct) is four times greater than the heat capacity of the target solution (Cs).

14. The apparatus recited in claim 1 wherein the means for shuttling (42) is configured to increase a pressure on the target solution during at least one phase of the thermal cycle so that the solution can be heated to a temperature higher than its atmospheric boiling point at that particular location.

15. The apparatus recited in claim 1, including a transparent coupler (16) having a side wall (16a), end walls (16b, 16c) which define an internal cavity (17), a first port (20) which receives a proximal end (12a) of the tube (12) and a second port (22) that extends radially through the side wall (16a), the coupler being fixed to a tube (12) illuminator (60) which directs light through the end wall (16a) axially into the proximal open end (12a) of the tube (12) such that light is propagated within the tube (12) illuminating the target solution.

16. The apparatus of claim 15 wherein the coupler (16) has a second port (22) connected to a pneumatic pressure source (42) and an inner cavity (17) in fluid communication between the pressure source (42) and the proximal end (12a) of the tube (12) such that the target solution is moveable within the tube (12) by the pressure source (42).

17. The apparatus of claim 1 wherein the means for shuttling (42) is configured such that the time to shuttle the target solution between each zone is 300 milliseconds.

18. The apparatus recited in any one of claims 1 to 17, wherein the distal end (12b) of the tube is resealable with the end cap (18).

## Patentansprüche

1. Vorrichtung zum Ausführen eines Verfahrens mit Polymerasekettenreaktion (PCR) an einer Säule einer Ziel-DNA-Lösung, umfassend:
a) ein längliches Rohr (12) mit einem proximalen Ende (12a), einem distalen Ende (12b), einer Längsachse, einer Denaturierungszone, einer Glühzone sowie einer Elongationszone;
b) ein Mittel zum Heizen der drei Heiz-/Kühlzonen (30a, 30b und 30c) bei unabhängigen erhöhten Temperaturen;
c) ein Mittel zum Vorwärts- und Rückwärtspendeln (42) der Ziellösung innerhalb des Rohres (12) von einer Zone zu einer anderen in einem sich wiederholenden thermischen Zyklus;
**dadurch gekennzeichnet, dass** sie umfasst:
d) ein Mittel zum Steuern (46) der Bewegung und der axialen Position der Ziellösung innerhalb des Rohres (12) während des thermischen Zyklus, mit einem Mittel zum Detektieren einer Position der Ziellösung in jeder Heiz-/Kühlzone,
und dass das distale Ende (12b) des länglichen Rohres mit einer entfernbaren Endkappe (18) abgedichtet ist.

2. Vorrichtung nach Anspruch 1, mit einem Mittel zum konduktiven und konvektiven Heizen/Kühlen (30) der Ziellösung in jeder Zone.

3. Vorrichtung nach einem der Ansprüche 1 oder 2, wobei das Verhältnis des Außendurchmessers des Rohres (12) zu dem Innendurchmesser des Rohres (12) größer als etwa 4 zu 1 ist.

4. Vorrichtung nach einem der Ansprüche 1 und 2, wobei das Heizmittel einen thermoelektrischen Heizblock (30) in jeder Zone umfasst, der das Rohr (12) relativ zu einer Arbeitsfläche in Kontakt bringt und trägt, und wobei jeder Block (30) eine längliche Nut (36) umfasst, in der das Rohr (12) eingesetzt ist.

5. Vorrichtung nach einem der Ansprüche 1 oder 2, wobei das Pendelmittel (42) ein Druckluftantriebsmittel (44) umfasst.

6. Vorrichtung nach Anspruch 5, wobei das Pendelmittel (42) einen Druckinjektor (42) umfasst, der eine wegwerfbare Spritze (48) aufweist, die in Fluidkommunikation mit einem Ende des Rohres (12) verbunden ist.

7. Vorrichtung nach einem der Ansprüche 1 oder 2, wobei das Steuermittel (46) ein Mittel zum Detektieren der Position der Ziellösung (60) in jeder Heiz-/Kühlzone aufweist.

8. Vorrichtung nach Anspruch 7, wobei das Positionsdetektionsmittel (60) einen Laseremitter (54) und einen Detektor (56) umfasst, die in einer Ausrichtung angeordnet sind, um einen Laserstrahl durch das Rohr (12) unter einem Winkel quer zu der Längsachse des Rohres (12) zu projizieren.

9. Vorrichtung nach Anspruch 8, mit einer Mehrzahl von Laseremittern (54) und Detektoren (56), die in Ausrichtungen angeordnet sind, die einen Laserstrahl durch jede Zone des Rohres (12) unter einem Winkel quer zu der Längsachse des Rohres (12) projizieren.

10. Vorrichtung nach einem der Ansprüche 1 oder 2, ferner mit einem Detektionsmittel (76), das das Verstärkungsniveau der Ziel-DNA während jedes thermischen Zyklus in Echtzeit detektiert.

11. Vorrichtung nach Anspruch 10, wobei jede der Denaturierungs-, Glüh- und Elongationszonen bei einer Temperatur gehalten werden, die eine PCR-Denaturierung, -Glühung bzw. -Elongation zulässt.

12. Vorrichtung nach Anspruch 11, wobei das Detektionsmittel eine Mehrzahl von optischen Fasern 76 umfasst, die in einer linearen Gruppierung in enger Nähe zu und in axialer Querausrichtung mit dem Rohr (12) in der Glühzone angeordnet sind.

13. Vorrichtung nach Anspruch 1, wobei das Material und die Abmessungen des Rohres (12) so gewählt sind, dass die Wärmekapazität der Wand des Rohres (12) pro Volumeneinheit (Ct) um das Vierfache größer als die Wärmekapazität der Ziellösung (Cs) ist.

14. Vorrichtung nach Anspruch 1, wobei das Mittel zum Pendeln (42) so konfiguriert ist, dass es einen Druck auf die Ziellösung während zumindest einer Phase des thermischen Zyklus erhöht, so dass die Lösung auf eine Temperatur erhitzt werden kann, die höher als ihr atmosphärischer Siedepunkt an dieser bestimmten Stelle ist.

15. Vorrichtung nach Anspruch 1, mit einem transparenten Koppler (16), der eine Seitenwand (16a), Endwände (16b, 16c), die einen Innenhohlraum (17) definieren, einen ersten Durchlass (20), der ein proximales Ende (12a) des Rohres (12) aufnimmt, und einen zweiten Durchlass (22) aufweist, der sich radial durch die Seitenwand (16a) erstreckt, wobei der Koppler an einer Beleuchtungseinrichtung (60) des Rohres (12) fixiert ist, die Licht durch die Endwand (16a) axial in das proximale offene Ende (12a) des Rohres (12) lenkt, so dass sich Licht in dem Rohr (12) ausbreitet, wobei die Ziellösung beleuchtet wird.

16. Vorrichtung nach Anspruch 15, wobei der Koppler (16) einen zweiten Durchlass (22), der mit einer Druckluftdruckquelle (42) verbunden ist, und einen Innenhohlraum (17) in Fluidkommunikation zwischen der Druckquelle (42) und dem proximalen Ende (12a) des Rohres (12) aufweist, so dass die Ziellösung in dem Rohr (12) durch die Druckquelle (42) bewegbar ist.

17. Vorrichtung nach Anspruch 1, wobei das Mittel zum Pendeln (42) so konfiguriert ist, dass die Zeit zum Pendeln der Ziellösung zwischen jeder Zone 300 Millisekunden beträgt.

18. Vorrichtung nach einem der Ansprüche 1 bis 17, wobei das distale Ende (12b) des Rohres mit der Endkappe (18) wieder abdichtbar ist.

## Revendications

1. Appareil pour exécuter une procédure de réaction en chaîne par polymérase (RCP) sur une colonne de solution d'ADN cible, comprenant :
a) un tube allongé (12) ayant une extrémité proximale (12a), une extrémité distale (12b), un axe longitudinal, une zone de dénaturation, une zone de températion, et une zone d'élongation ;
b) des moyens pour chauffer desdites trois zones de chauffage/refroidissement (30a, 30b et 30c) à des températures élevées indépendantes ;
c) un moyen pour déplacer (42) la solution cible en va-et-vient à l'intérieur dudit tube (12) depuis une zone à une autre dans un cycle thermique répété ;
**caractérisé en ce qu'**il inclut :
d) un moyen pour commander (46) le mouvement et la position axiale de la solution cible à l'intérieur dudit tube (12) pendant le cycle thermique, incluant un moyen pour détecter une position de la solution cible à l'intérieur de chacune desdites zones de chauffage/refroidissement,
et **en ce que** l'extrémité distale (12b) dudit tube allongé est scellée avec un capuchon d'extrémité amovible (18).

2. Appareil selon la revendication 1, incluant un moyen pour chauffer/refroidir (30) la solution cible par conduction et par convection dans chaque zone.

3. Appareil selon l'une quelconque des revendications 1 et 2, dans lequel le rapport du diamètre extérieur du tube (12) sur le diamètre intérieur du tube (12) est supérieur à environ 4:1.

4. Appareil selon l'une quelconque des revendications 1 et 2, dans lequel ledit moyen de chauffage comprend un bloc de chauffage thermoélectrique (30) dans chaque zone, qui est en contact avec et qui supporte le tube (12) par rapport à une surface de travail, et dans lequel chaque bloc (30) inclut une gorge allongée (36) dans laquelle le tube (12) est logé.

5. Appareil selon l'une quelconque des revendications 1 et 2, dans lequel ledit moyen de déplacement en va-et-vient (42) comprend un moyen d'entraînement pneumatique (44).

6. Appareil selon la revendication 5, dans lequel ledit moyen de déplacement en va-et-vient (42) comprend un injecteur de pression (42) incluant une seringue à jeter (48) connectée en communication fluidique à une extrémité dudit tube (12).

7. Appareil selon l'une quelconque des revendications 1 et 2, dans lequel ledit moyen de commande (46) inclut un moyen pour détecter la position de la solution cible (60) à l'intérieur de chaque zone de chauffage/refroidissement.

8. Appareil selon la revendication 7, dans lequel ledit moyen de détection de position (60) comprend un émetteur à laser (54) et un détecteur (56) agencés dans un alignement afin de de projeter un faisceau laser à travers le tube (12) sous un angle transversal à l'axe longitudinal dudit tube (12).

9. Appareil selon la revendication 8, incluant une pluralité d'émetteurs à laser (54) et de détecteurs (56) agencés dans des alignements, qui projettent un faisceau laser à travers de chaque zone du tube (12) sous un angle transversal à l'axe longitudinal du tube (12).

10. Appareil selon l'une quelconque des revendications 1 et 2, incluant en outre un moyen de détection (76) qui détecte le niveau d'amplification de l'ADN cible pendant chaque cycle thermique en temps réel.

11. Appareil selon la revendication 10, dans lequel chacune de la zone de dénaturation, de la zone de températion et de la zone d'élongation est maintenue à une température qui permet une dénaturation RCP, une températion et une élongation, respectivement.

12. Appareil selon la revendication 11, dans lequel ledit moyen de détection comprend une pluralité de fibres optiques (76) agencées dans un réseau linéaire à proximité de et en alignement axialement transversal avec le tube (12) dans la zone de températion.

13. Appareil selon la revendication 1, dans lequel le matériau et les dimensions du tube (12) sont sélectionnés de telle façon que la capacité thermique de la paroi du tube (12) par volume unitaire (Ct) est quatre fois plus élevée que la capacité thermique de la solution cible (Cs).

14. Appareil selon la revendication 1, dans lequel le moyen de déplacement en va-et-vient (42) est configuré pour augmenter une pression sur la solution cible pendant au moins une phase du cycle thermique, de telle façon que la solution peut être chauffée à une température plus élevée que son point d'ébullition atmosphérique à cet emplacement particulier.

15. Appareil selon la revendication 1, incluant un coupleur transparent (16) ayant une paroi latérale (16a), des parois terminales (16b, 16c) qui définissent une cavité interne (17), un premier orifice (20) qui reçoit une extrémité proximale (12a) du tube (12) et un second orifice (22) qui s'étend radialement à travers la paroi latérale (16a), le coupleur étant fixé à un moyen d'illumination (60) du tube (12) qui dirige de la lumière à travers la paroi terminale (16a) axialement dans l'extrémité ouverte proximale (12a) du tube (12) de sorte que la lumière se propage à l'intérieur du tube (12) en illuminant la solution cible.

16. Appareil selon la revendication 15, dans lequel le coupleur (16) a un second orifice (22) connecté à une source de pression pneumatique (42) et une cavité intérieure (17) en communication fluidique entre la source de pression (42) et l'extrémité proximale (12a) du tube (12) de sorte que la solution cible est déplaçable à l'intérieur du tube (12) par la source de pression (42).

17. Appareil selon la revendication 1, dans lequel le moyen de déplacement en va-et-vient (42) est configuré de telle façon que le temps pour déplacer la solution cible en va-et-vient entre chaque zone est de 300 ms.

18. Appareil selon l'une quelconque des revendications 1 à 17, dans lequel l'extrémité distale (12b) du tube est susceptible d'être rescellée avec le capuchon d'extrémité (18).
